# EUROPEAN PATENT SPECIFICATION

(11) **EP 2 719 936 B1**
(45) Date of publication and mention of the grant of the patent: **22.06.2016**
(21) Application number: 12801080.8
(22) Date of filing: 07.05.2012
(51) Int. Cl.: F16L 55/00, A61M 25/09

(54) **RESIN TUBE FOR GUIDE WIRE, METHOD FOR MANUFACTURING RESIN TUBE FOR GUIDE WIRE, AND GUIDE WIRE**
KUNSTHARZROHR FÜR EINEN FÜHRUNGSDRAHT, VERFAHREN ZUR HERSTELLUNG DES KUNSTHARZROHRS FÜR EINEN FÜHRUNGSDRAHT UND FÜHRUNGSDRAHT
TUBE EN RÉSINE POUR FIL DE GUIDAGE, PROCÉDÉ DE FABRICATION D'UN TUBE EN RÉSINE POUR FIL DE GUIDAGE, ET FIL DE GUIDAGE

(30) Priority: 13.06.2011 JP 2011131168
(43) Date of publication of application: 16.04.2014
(73) Proprietor: Nissei Electric Co., Ltd., Shizuoka 432-8006 (JP)
(72) Inventor: NAKAJIMA Tetsuya, Hamamatsu-shi Shizuoka 432-8006 (JP); KIKUCHI Hideki, Hamamatsu-shi Shizuoka 432-8006 (JP)
(74) Representative: Epping - Hermann - Fischer
(86) International application number: PCT/JP2012/061674
(87) International publication number: WO 2012/172881

(56) References cited:
- EP-A2- 1 243 283
- WO-A1-2009/004876
- JP-A- 8 326 959
- JP-A- 62 231 675
- JP-A- 2008 155 054
- JP-B1- 4 620 177
- US-A1- 2004 215 109
- US-A1- 2004 215 109

## Description

### Technical Field

The present invention relates to resin tube for a guide wire to be inserted into a pipe, a method for manufacturing a resin tube for a guide wire, and a guide wire.

### Background Art

Conventionally, guide wires are used in order to clean, inspect, or lay wires through narrow pipes such as plumbing, gas, electrical, and automotive piping or the like. Also, in the medical field, medical guide wires inserted into blood vessels, ureters, or organs for treatment or inspection are used.

In the case of drawing a long guide wire through a narrow site such as piping, with a guide wire having a simple circular cross-section, the surface is smooth and the contact area with the insertion site increases, and the sliding properties worsen. Thus, problems readily occur, such as work taking time, or the guide wire itself or the insertion site being damaged during work. Accordingly, sliding properties are being improved by using fluoropolymers with excellent friction reducing properties, such as polytetrafluoroethylene (hereinafter, PTFE), but the improvement is still insufficient.

Thus, there exists a guide wire like that illustrated in Patent Literature 1, for example. The guide wire in Patent Literature 1 has a core wire with a reduced-diameter tip. An unevenness is provided on the outer circumference of the core wire, and a resin film is provided surrounding the core wire. Additionally, when inserting the guide wire into a pipe, the convex portions of the unevenness of the resin film formed by the unevenness of the core wire partially contact the inner circumference of the pipe, and thus sliding resistance between the guide wire and the pipe is reduced, enabling the guide wire to move smoothly.

Also, the guide wire illustrated in Patent Literature 2 consists of a wire having a length extending along a long axis and a rectangular cross-section, whose circumference on the long axis is twisted in a helical shape.

Additionally, with the guide wire illustrated in Patent Literature 3, the outer circumference of a core wire is covered with a resin film at least partially having an unevenly shaped helical pattern that is color-coded with multiple colors.

Additionally, the guide wire illustrated in Patent Literature 4 is a medical guide wire made of plastic, and consists of a long main section. A flexible wire is inserted into an inner core section ofthe main section, and a concave surface section drilled with grooves for decreasing the contact area is formed on the outer surface of the main section.

JP 4620177 discloses a resin tube according to the preamble of claims 1 and 5, a method according to the preamble of claim 9.

### Patent Literature

Patent Literature 1: International Publication Pamphlet No. WO 2009/004876
Patent Literature 2: United States Patent Application Publication No. 2004/0215109
Patent Literature 3: Unexamined Japanese Patent Application Kokai Publication No. 2007-97662
Patent Literature 4: Unexamined Japanese Patent Application Kokai Publication No. S62-231675

### Summary of Invention

### Technical Problem

However, with the guide wires of the past, if the core wire is covered with a resin tube in the case where an unevenness is provided on the outer circumference of the core wire, the unevenness is less likely to be expressed on the surface of the guide wire itself. Meanwhile, with an unevenness merely due to a helical pattern, it is difficult to ensure sufficient sliding properties with respect to the object of contact at the work site (inside a pipe, for example). Also, although a structure such as one in which grooves are drilled may have given sliding properties, it is difficult to prevent kinking and collapsing, and improve buckling resistance.

The present invention has been devised in light of such circumstances, and provides a resin tube for a guide wire, a method for manufacturing a resin tube for a guide wire, and a guide wire that makes point contact in the axial direction with an object of contact at a work site, thereby reducing frictional resistance and yielding excellent sliding properties, while also being prevented from kinking and collapsing, and having excellent buckling resistance.

### Solution to Problem

A resin tube for a guide wire according to Claim 1 is characterized in being twisted such that the length of the pitch, being the distance in the axial direction over which one point on the outer circumference of a cross-sectional outer shape of the trunk section is twisted 360 degrees in a helical shape and returns to the original position, becomes irregular in the axial direction of the trunk section.

The resin tube for a guide wire according to Claim 2 is characterized in that the height of a ridge line extending in the axial direction of the trunk section varies along the axial direction.

The resin tube for a guide wire according to Claim 3 is characterized in being twisted such that the length of the pitch, being the distance in the axial direction over which one point on the outer circumference of a cross-sectional outer shape of the trunk section is twisted 360 degrees in a helical shape and returns to the original position, is 6 times to 160 times a maximum radius R1, being the distance from the axis of twisting to a farthest point on the cross-sectional outer shape.

The resin tube for a guide wire according to Claim 5 is characterized in that the relationship between a maximum radius R1, being the distance from the axis of twisting to a farthest point on the cross-sectional outer shape, and a minimum radius R2, being the distance from the axis of twisting to a nearest point on the cross-sectional outer shape, is 0.4 ≤ R2 / R1 ≤ 0.9.

A method for manufacturing a resin tube for a guide wire according to Claim 9 is characterized by twisting the extruded tube in a helical shape by an irregular rotational.

The method for manufacturing a resin tube for a guide wire according to Claim 10 is characterized by twisting the extruded tube by a rotational amount such that the length of the pitch, being the distance in the axial direction over which one point on the outer circumference of the cross-sectional outer shape is twisted 360 degrees in a helical shape and returns to the original position, is 6 times to 160 times a maximum radius R1, being the distance from the axis of twisting to a farthest point on the cross-sectional outer shape.

A guide wire according Claim 11 is characterized by comprising a resin tube that has a cross-sectional outer shape perpendicular to an axial direction that is polygonal, elliptic, or irregular, is twisted in a helical shape about a deep inner-core hole extending in the axial direction as an axis of twisting, and includes a trunk section having an undulation on the outer surface thereof caused by the twisted eccentric wall thickness, and a core wire inserted into the deep inner-core hole of the resin tube.

The guide wire according to Claim 12 is characterized in that the height of a ridge line extending in the axial direction of the trunk section of the resin tube varies along the axial direction.

The guide wire according to Claim 13 is characterized in that the resin tube is twisted such that the length of the pitch, being the distance in the axial direction over which one point on the outer circumference of a cross-sectional outer shape of the trunk section of the resin tube is twisted 360 degrees in a helical shape and returns to the original position, is 6 times to 160 times a maximum radius R1, being the distance from the axis of twisting to a farthest point on the cross-sectional outer shape.

The guide wire according to Claim 14 is characterized in that the resin tube is twisted such that the length of the pitch, being the distance in the axial direction over which one point on the outer circumference of a cross-sectional outer shape of the trunk section of the resin tube is twisted 360 degrees in a helical shape and returns to the original position, becomes irregular in the axial direction of the trunk section.

The guide wire according to Claim 15 is characterized in that the relationship between a maximum radius R1, being the distance from the axis of twisting to a farthest point on the cross-sectional outer shape of the trunk section of the resin tube, and a minimum radius R2, being the distance from the axis of twisting to a nearest point on the cross-sectional outer shape, is 0.4 ≤ R2 / R1 ≤ 0.9.

### Advantageous Effects of Invention

According to the present invention, point contact is made in the axial direction with an object of contact at a work site, thereby reducing frictional resistance and yielding excellent sliding properties, while also preventing kinking and collapsing, and yielding excellent buckling resistance.

### Brief Description of Drawings

FIG. 1A is an exterior view illustrating an example of a resin tube according to the present invention;
FIG. 1B is a side view illustrating an example of a resin tube according to the present invention;
FIG. 1C is an end view illustrating an example of a resin tube according to the present invention;
FIG. 2A is an exterior view illustrating another example of a resin tube according to the present invention;
FIG. 2B is an end view illustrating another example of a resin tube according to the present invention;
FIG. 3A is an exterior explanatory diagram for the resin tube in FIG. 2;
FIG. 3B is an exterior explanatory diagram for the resin tube in FIG. 2;
FIG. 3C is a cross-sectional explanatory diagram for the resin tube in FIG. 2;
FIG. 4 is an end view illustrating yet another example of a resin tube according to the present invention;

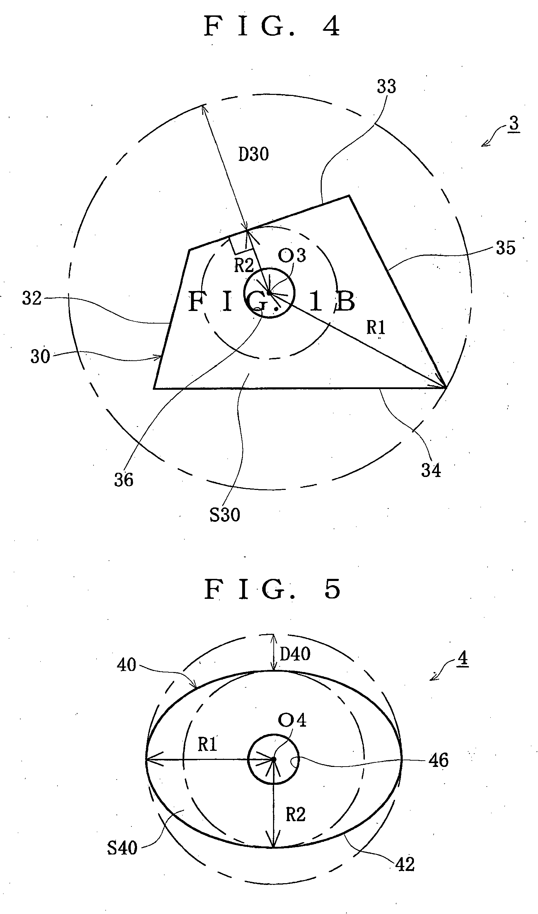

FIG. 4 is an end view illustrating yet another example of a resin tube according to the present invention; and
FIG. 5 is an end view illustrating yet another example of a resin tube according to the present invention.

### Description of Embodiments

A guide wire of the present embodiment is equipped with a resin tube having a trunk section through which a deep inner-core hole extending in the axial direction is drilled, and a core wire inserted into the deep inner-core hole of the resin tube. In other words, the guide wire is configured such that the core wire is covered by the resin tube. Since the guide wire is used in order to clean, inspect, or lay wires through narrow pipes such as plumbing, gas, electrical, and automotive piping or the like, a long guide wire is used by being drawn through a narrow site such as piping. The field of use (usage method) of a guide wire of the present embodiment may be, for example, cleaning of cylindrical objects such as water pipes, gas pipes, and manholes, piping inspection, laying wiring such as automotive, electrical, or telephone wires through narrow pipes, or the like. Also, in the medical field, a guide wire inserted into blood vessels, ureters, or organs for treatment or inspection may be used.

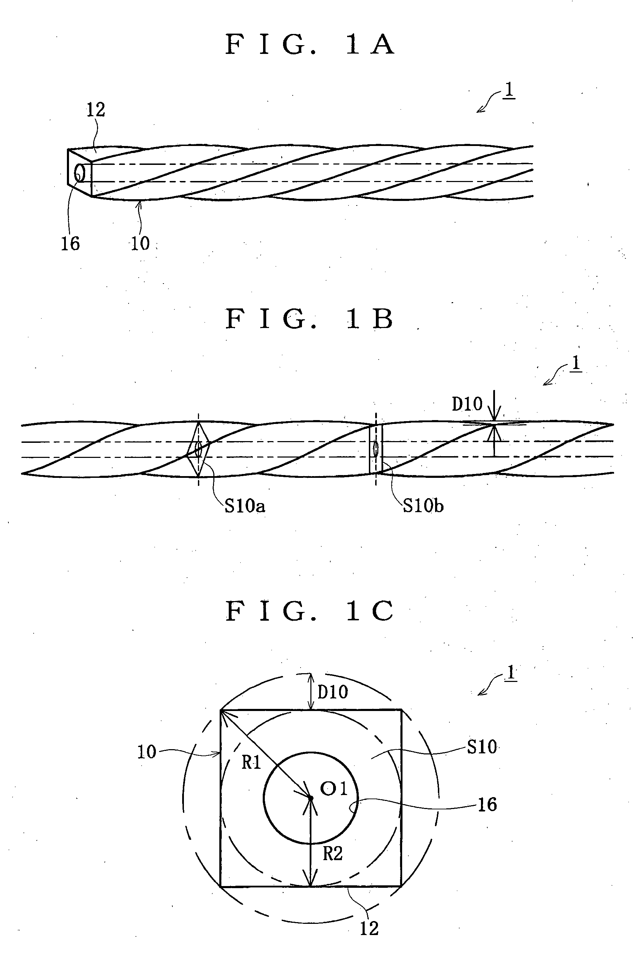

Hereinafter, a guide wire of the present embodiment will be described with reference to the attached drawings. In particular, an embodiment of the resin tube from among the components of a guide wire will be described. FIG. 1A is an exterior view illustrating an example of a resin tube according to the present invention. FIG. 1B is a side view illustrating an example of a resin tube according to the present invention. FIG. 1C is an end view illustrating an example of a resin tube according to the present invention. The resin tube 1 illustrated in FIGS. 1A to 1C is a long resinous extrusion, and is a tube for a guide wire consisting of a trunk section 10 in which is drilled a deep inner-core hole 16 extending in the axial direction and allowing insertion of a core wire to be discussed later. In the resin tube 1, the outer shape (cross-sectional outer shape) of a cross-section S10 (S10a, S10b) perpendicular to the axial direction is square (FIG. 1C). The resin tube 1 is twisted in a helical shape about the deep inner-core hole 16 as the axis of twisting O1, and has an undulation on the outer surface 12 of the trunk section 10 caused by the twisted eccentric wall thickness.

Also, the resin tube 1 is twisted such that the length of the pitch, which is the distance in the axial direction over which one point on the outer circumference of a cross-sectional outer shape perpendicular to the axial direction of the trunk section 10 is twisted 360 degrees in a helical shape and returns to the original position, is 6 times to 160 times the maximum radius R1, which is the distance from the axis of twisting O1 to a farthest point on a cross-sectional outer shape. Herein, the maximum radius R1 of the resin tube 1 is the distance from the axis of twisting O1 to a vertex of a square, as illustrated in FIG. 1C.

Furthermore, in the resin tube 1, the relationship between the maximum radius R1, which is the distance from the axis of twisting O1 to a farthest point on a cross-sectional outer shape, and a minimum radius R2, which is the distance from the axis of twisting O1 to a nearest point on a cross-sectional outer shape, is 0.4 ≤ R2 / R1 ≤ 0.9. Herein, the minimum radius R2 of the resin tube 1 is the distance from the axis of twisting O1 to the outer surface 12 when drawing a normal line from the axis of twisting O1 to the outer surface 12, as illustrated in FIG. 1C.

The resin tube 1 is manufactured by extruding resin into a tube with a square cross-sectional outer shape perpendicular to the axial direction and having a deep inner-core hole 16 extending in the axial direction and allowing a core wire to be inserted therein. After that, the extruded tube is set in a helix processing machine, and twisted by a given rotational amount about the deep inner-core hole 16 as the axis of twisting O1 under high temperature. In this way, since the resin tube 1 is twisted after first being extruded into a long tube shape with the same cross-sectional outer shape in the axial direction, the cross-sectional outer shapes (cross-sections S10a, S10b) perpendicular to the axial direction of the twisted resin tube 1 are approximately the same at all positions, as illustrated in FIG. 1B. Note that the twisting in this manufacturing process is such that the length of the pitch becomes 6 times to 160 times the maximum radius R1. The resin tubes 2 to 4 discussed later have a similar manufacturing method. Also, by inserting a core wire into the deep inner-core holes 16,26,36, and 46 of the resin tubes 1 to 4 manufactured according to this method, a guide wire is completed. Note that the core wire is not limited in material, function, or the like, insofar as it may be used as a guide wire.

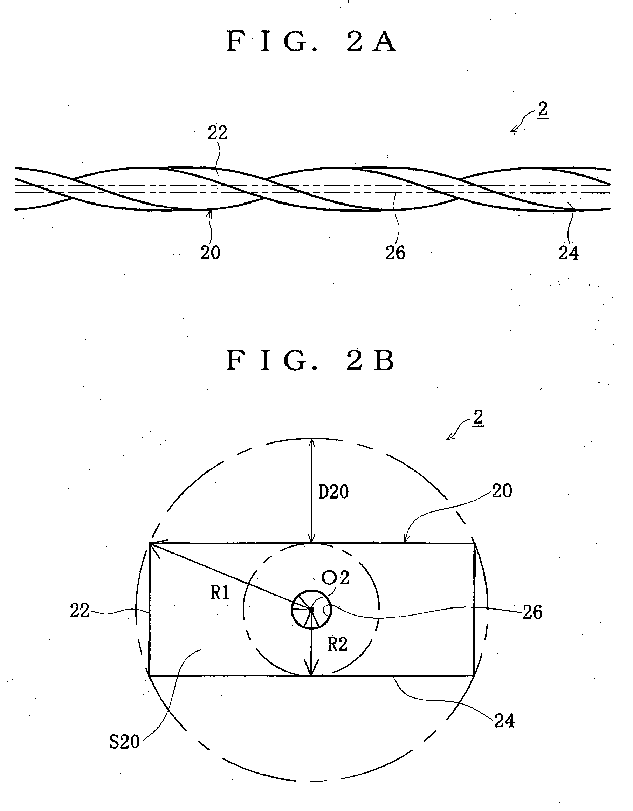

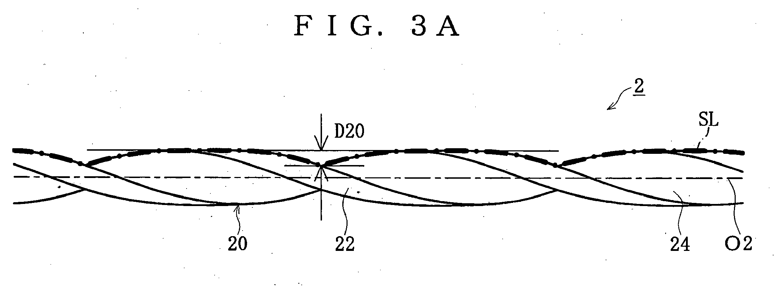

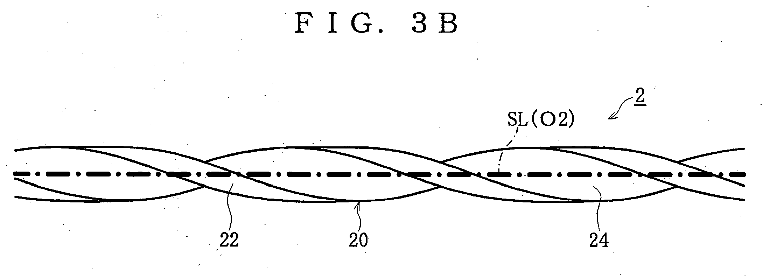

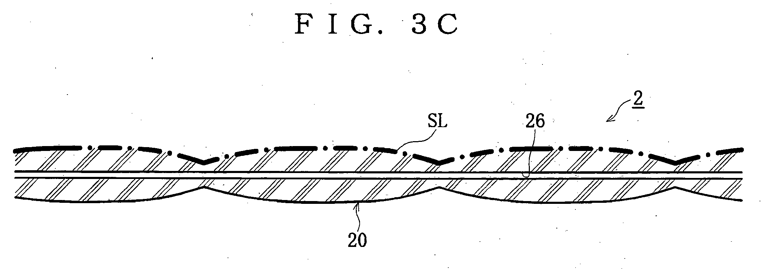

Although the cross-sectional outer shape perpendicular to the axial direction of the above resin tube 1 is square, examples of polygonal shapes include examples like those illustrated in FIGS. 2 and 3 as well as in FIG. 4. FIG. 2A is an exterior view illustrating another example of a resin tube according to the present invention. FIG. 2B is an end view illustrating another example of a resin tube according to the present invention. FIG. 3A is an exterior explanatory diagram for the resin tube in FIG. 2. FIG. 3B is an exterior explanatory diagram for the resin tube in FIG. 2. FIG. 3C is a cross-sectional explanatory diagram for the resin tube in FIG. 2.

Similarly to the resin tube 1, the resin tube 2 in FIGS. 2 and 3 is a long resinous extrusion, and is a tube for a guide wire consisting of a trunk section 20 in which is drilled a deep inner-core hole 26 extending in the axial direction and allowing the insertion of a core wire to be discussed later. In the resin tube 2, the outer shape (cross-sectional outer shape) of a cross-section S20 perpendicular to the axial direction is rectangular (FIG. 2B). The resin tube 2 is twisted in a helical shape about the deep inner-core hole 26 as the axis of twisting 02, and has an undulation on the outer surfaces 22 and 24 of the trunk section 20 caused by the twisted eccentric wall thickness.

Also, similarly to the resin tube 1, the resin tube 2 is twisted such that the length of the pitch, which is the distance in the axial direction over which one point on the outer circumference of a cross-sectional outer shape perpendicular to the axial direction ofthe trunk section 20 is twisted 360 degrees in a helical shape and returns to the original position, is 6 times to 160 times the maximum radius R1, which is the distance from the axis of twisting 02 to a farthest point on a cross-sectional outer shape. Herein, the maximum radius R1 of the resin tube 2 is the distance from the axis of twisting 02 to a vertex of a rectangle, as illustrated in FIG. 2B.

Furthermore, similarly to the resin tube 1, in the resin tube 2, the relationship between the maximum radius R1, which is the distance from the axis of twisting 02 to a farthest point on a cross-sectional outer shape, and a minimum radius R2, which is the distance from the axis of twisting 02 to a nearest point on a cross-sectional outer shape, is 0.4 ≤ R2 / R1 ≤ 0.9. Herein, the minimum radius R2 of the resin tube 2 is the distance from the axis of twisting 02 to the outer surface 24 when drawing a normal line from the axis of twisting 02 to the outer surface 24, as illustrated in FIG. 2B.

Note that in the resin tube 2, and similarly to the resin tube 1 as well as the resin tubes 3 and 4 discussed later, the height of a ridge line SL extending in the axial direction of the trunk section 20 varies along the axial direction. Herein, the ridge line SL in this specification refers to a line on the outer surfaces 22 and 24 extending straight and parallel to the axis of twisting O2. If described using the drawings, when the resin tube 2 is viewed from the side, as illustrated in FIG. 3A, the ridge line SL is the line that forms the contour at the top of the trunk section 20. Also, if illustrated from a position viewed from above, as in FIG. 3B, the ridge line SL in FIG. 3A appears overlapping the axis of twisting 02. Note that since the ridge line is a line indicating a contour, the bottom contour in FIG. 3A may also be referred to as a ridge line, and multiple ridge lines exist depending on the position from which the resin tube 2 is viewed.

Additionally, as illustrated in FIG. 3A, the height of the ridge line SL varies along the axial direction, and the portion with the largest such height difference is the maximum height difference D20. This maximum height difference D20 is the difference between the maximum radius R1 and the minimum radius R2 illustrated in FIG. 2B. This applies similarly to the resin tube 1, and the maximum height difference D10 of a ridge line on the resin tube 1 (FIG. 1B) is the difference between the maximum radius R1 and the minimum radius R2 illustrated in FIG. 1C.

In this way, the resin tube 1 and the resin tube 2 are twisted in a helical shape about the deep inner-core holes 16 and 26 as axes.of twisting O1 and 02, with the wall thickness with respect to the deep inner-core holes 16 and 26 being eccentric. Furthermore, due to being twisted, an undulation is produced in the outer surfaces 12,22, and 24 of the trunk sections 10 and 20 caused the eccentric wall thickness. Additionally, the height of a ridge line SL, which is a contour viewed from the side of the resin tube 1 and the resin tube 2, varies along the axial direction. Due to such an undulation (the variance in the height of the ridge line SL along the axial direction), point contact is made in the axial direction with an object of contact at a work site, thereby reducing frictional resistance and yielding excellent sliding properties. Note that this advantageous effect due to the undulation of the outer surfaces 12, 22, and 24 or the variance in the height of the ridge line SL is also similar for the resin tubes 3 and 4 discussed later.

Although the outer shapes (cross-sectional outer shapes) of the cross-sections S10 (S10a, S10b) and S20 perpendicular to the axial direction of the above resin tube 1 and resin tube 2 are square or rectangular, a many-sided regular polygon or irregular polygon is also acceptable. Examples of an irregular polygon include the resin tube 3 illustrated in FIG. 4, for example. In the resin tube 3 illustrated in FIG. 4, the outer shape (cross-sectional outer shape) of a cross-section S30 perpendicular to the axial direction is an irregular polygon. The outer shape of the cross-section S30 is formed by outer surfaces 32, 33, 34, and 35 with respectively different edge lengths, and the angles of the respective corners also differ. In addition, the resin tube 3 is twisted in a helical shape about a deep inner-core hole 36 as the axis of twisting 03, has an undulation on the outer surfaces 32, 33, 34, and 35 of the trunk section 30 caused by the twisted eccentric wall thickness.

In addition, similarly to the resin tube 1 and the resin tube 2, the resin tube 3 is twisted such that the length of the pitch, which is the distance in the axial direction over which one point on the outer circumference of a cross-sectional outer shape perpendicular to the axial direction of the trunk section 30 is twisted 360 degrees in a helical shape and returns to the original position, is 6 times to 160 times the maximum radius R1, which is the distance from the axis of twisting 03 to a farthest point on a cross-sectional outer shape. Also, the relationship between the maximum radius R1, which is the distance from the axis of twisting 03 to a farthest point on a cross-sectional outer shape, and a minimum radius R2, which is the distance from the axis of twisting 03 to a nearest point on a cross-sectional outer shape, is 0.4 ≤ R2 / R1 ≤ 0.9, similarly to the resin tube 1 and the resin tube 2.

Herein, the maximum radius R1 of the resin tube 3 is the distance to the position farthest away from the axis of twisting 03 on the outer circumference of the cross-section S30, and thus, as illustrated in FIG. 4, becomes the distance to the vertex of the corner whose position is separated the farthest distance away from the axis of twisting 03 (the corner formed by the outer surface 34 and the outer surface 35). Also, the minimum radius R2 of the resin tube 3 is the distance to the position nearest the axis of twisting 03 on the outer circumference of the cross-section S30, and thus, as illustrated in FIG. 4, is the distance to the edge at the closest distance to the axis of twisting 03, or in other words, the distance to the outer surface 33 when drawing a normal line from the axis of twisting 03. In the resin tube 1, the outer shape of the cross-section S10 is square, and the height of the undulation is comparatively constant, but in the case of the resin tube 3, large, large, small, small becomes a series for one period, and in the case of an irregular shape becomes a period with more complex variation in the undulation.

Furthermore, the outer shape (cross-sectional outer shape) of a cross-section perpendicular to the axial direction of a resin tube in this specification is not limited to a polygonal shape, and also includes the resin tube 4 illustrated in FIG. 5, for example. In the resin tube 4 illustrated in FIG. 5, the outer shape (cross-sectional outer shape) of a cross-section S40 perpendicular to the axial direction is elliptic. In addition, the resin tube 4 is twisted in a helical shape about a deep inner-core hole 46 as the axis of twisting 04, and has an undulation on the outer surface 42 of the trunk section 40 caused by the twisted eccentric wall thickness.

Similarly to the resin tubes 1 to 3, the resin tube 4 is twisted such that the length of the pitch, which is the distance in the axial direction over which one point on the outer circumference of a cross-sectional outer shape perpendicular to the axial direction ofthe trunk section 40 is twisted 360 degrees in a helical shape and returns to the original position, is 6 times to 160 times the maximum radius R1, which is the distance from the axis oftwisting 04 to a farthest point on a cross-sectional outer shape. Also, the relationship between the maximum radius R1, which is the distance from the axis of twisting O4 to a farthest point on a cross-sectional outer shape, and a minimum radius R2, which is the distance from the axis of twisting 04 to a nearest point on a cross-sectional outer shape, is 0.4 ≤ R2 / R1 ≤ 0.9, similarly to the resin tubes 1 to 3.

Herein, the maximum radius R1 of the resin tube 4 is the distance to the position farthest away from the axis of twisting 04 on the outer circumference of the cross-section S40, and thus, as illustrated in FIG. 5, becomes the distance to the long edge position separated the farthest distance away from the axis of twisting 03. Also, the minimum radius R2 is the distance to the position nearest the axis oftwisting 04 on the outer circumference ofthe cross-section S40, and thus, as illustrated in FIG. 5, is the distance to the short edge position at the closest distance to the axis of twisting 04.

Although the outer shape of the cross-section S40 of the resin tube 4 illustrated in FIG. 5 is an ellipse with no distortion, an ellipse with distortion on the outer circumference is also acceptable, and is not limited to the cross-sectional outer shape illustrated by the cross-section S40.

Note that the above resin tubes 1 to 4 merely illustrate examples of the outer shape of a cross-section, and the cross-sectional outer shape perpendicular to the axial direction (the cross-section perpendicular to the axial direction has the same shape when viewed at any position in the axial direction), although preferably polygonal, is not particularly limited insofar as the cross-sectional outer shape is an irregular shape such as an ellipse or star shape (including combinations of polygonal and elliptic shapes) and has a difference between the maximum radius R1 and the minimum radius R2 of the cross-sectional outer shape. In the case where the cross-sectional outer shape is polygonal, the shape of the polygon is not particularly limited. Although a triangle, quadrilateral, or the like has few corners, the unevenness of the ridge line section becomes acute, and the unevenness is easily recognizable. Also, a regular polygon such as a triangle or square is not strictly necessary. For example, besides an isosceles triangle or rectangle, for example, the lengths of each edge may additionally all differ, and crooked polygons that are not symmetrical are also acceptable. Also, the corner sections of a polygon may be acute, obtuse, or rounded. In addition to polygonal, elliptic, and irregular shapes, an uneven shape having multiple gear-shaped cogs is also acceptable, but in consideration of strength as a resin tube, a shape having cogs is not preferable.

The shape of the deep inner-core hole is circular or polygonal, and is not particularly limited.

Furthermore, the material of the resin tubes 1 to 4 may be a fluoropolymer such as PTFE, polyolefin, a thermoplastic such as nylon, or a thermoplastic elastomer preferable as a guide wire, for example. In consideration of sliding properties, polytetrafluoroethylene (PTFE) is particularly preferable. Note that the material is not limited to these materials, and may be appropriately selected according to the application insofar as the material is a resin. Also, the resin tubes 1 to 4 include those in which the resin tubes 1 to 4 are enlarged or constricted in diameter. Furthermore, by roughening the outer surface of the resin tubes 1 to 4 with a sandblasting process or a rolling process, it is also possible to further vary the undulation.

The length of the pitch is preferably 6 times to 160 times the maximum radius R1. When specifically illustrated in terms of the example of the resin tube 1 whose cross-sectional outer shape is square, in the case where the maximum radius R1 is 0.12 mm, for example, the length of the pitch is 33 times to 133 times the maximum radius R1. Also, in the case where the maximum radius R1 is 0.42 mm, the length of the pitch is 10 times to 38 times the maximum radius R1. Furthermore, in the case where the maximum radius R1 is 4.25 mm, the length of the pitch is 16 times to 66 times the maximum radius R1. Differences in advantageous effects with respect to the relationship between the length of the pitch and the maximum radius R1 are illustrated in Table 1.

**Table 1.**

| Pitch (multiple of R1) | Unevenness | Kinking, Collapsing | Productivity |
|---|---|---|---|
| less than 6 | A | D | D |
| 6 to less than 9 | A | C | C |
| 9 to less than 140 | B | B | B |
| 140 to 160 | C | A | A |
| over 160 | D | A | A |

As illustrated in Table 1, in the case where the length of the pitch is less than 6 times the maximum radius R1, the unevenness (undulation) of the outer surface is greatly expressed and yields excellent sliding properties, but kinking occurs, problems such as collapsing of the deep inner-core holes 16, 26, 36, and 46, and bending (buckling) during work readily occur, and the complications of production also lower productivity. In the case where the length of the pitch is from 6 times to less than 9 times the maximum radius R1, there is a risk of kinking, collapsing, and the like, and productivity is also lowered somewhat, but unevenness (undulation) is greatly expressed to yield excellent sliding properties, and practical use is possible. In the case where the length of the pitch is from 9 times to less than 140 times the maximum radius R1, sliding properties due to unevenness (undulation) is sufficiently ensured, there is also little worry of kinking, collapsing, or the like, and there is stable productivity. The pitch of which the length of the pitch is from 9 times to less than 140 times the maximum radius R1 is the most practical range. In the case where the length of the pitch is from 140 times to 160 times the maximum radius R1, there is no risk of kinking, collapsing, or the like, and productivity is also excellent, and although the sliding properties are slightly lowered due to the mild unevenness (undulation), practical use is possible. In the case where the length of the pitch exceeds 160 times the maximum radius R1, there is no risk of kinking, collapsing, or the like, and productivity is also excellent, but the sliding properties are lowered due to the extremely mild unevenness (undulation), and sufficient advantageous effects cannot be expected. Given the above, it may be said that it is preferable to twist such that the length of the pitch becomes 6 times to 160 times the maximum radius R1.

Note that a resin tube may also be twisted such that the length of the pitch becomes irregular in the axial direction ofthe trunk sections 10, 20, 30, and 40. In other words, the configuration is not limited by the periodicity of the pitch. Also, it is sufficient to appropriately set the twisting amount, or in other words the rotational amount, of the trunk sections 10, 20, 30, and 40 with a rotational angle, rotational speed, or the like to so as to yield the above length of the pitch. By making the length of the pitch irregular, contact with an object of contact at a work site (inside a pipe, for example) becomes irregular point contact, and it is possible to further improve the sliding properties compared to regular point contact.

The relationship between the maximum radius R1 and the minimum radius R2 is preferably 0.4 ≤ R2 / R1 ≤ 0.9. Specifically, differences in advantageous effects with respect to the relationship between the maximum radius R1 and the minimum radius R2 are illustrated in Table 2.

**Table 2.**

| R2 / R1 | Buckling Resistance | Unevenness |
|---|---|---|
| 1.0 | A | D |
| 0.9 | A | C |
| 0.8 | B | B |
| 0.7 | B | B |
| 0.6 | B | B |
| 0.5 | B | B |
| 0.4 | C | A |
| 0.3 | D | A |
| 0.2 | D | A |
| 0.1 | D | A |

As illustrated in Table 2, in the case where minimum radius R2 / maximum radius R1 is 1.0, there is no difference between the maximum radius R1 and the minimum radius R2, an unevenness (undulation) is not produced on the outer surface, and although buckling is less likely, the sliding properties are lowered. For this reason, 1.0 is not a preferable ratio. In contrast, for a minimum radius R2 / maximum radius R1 from 0.9 to 0.4, a sufficient unevenness (undulation) is produced on the outer surface to yield excellent sliding properties, while buckling is also less likely and the resin tube is easy to work with. Note that in the case where minimum radius R2 / maximum radius R1 is less than 0.4, a sufficient unevenness (undulation) is produced on the outer surface to yield excellent sliding properties, but the difference in the thickness of the trunk section (the maximum height difference) is extremely large, buckling is likely during work, and adverse effects such as low rigidity and difficulty in maintaining shape are produced. Given the above, it may be said that the trunk sections 10, 20, 30, and 40 are preferably extruded such that the relationship between the maximum radius R1 and the minimum radius R2 becomes 0.4 ≤ R2 / R1 ≤ 0.9.

Various embodiments and examples of the present invention are possible without departing from the scope and spirit of the present invention in the broad sense. Also, the embodiments and examples discussed above are for the purpose of describing the present invention, and do not limit the scope of the present invention.

### Industrial Applicability

As above, since a resin tube may be exxtruded with an undulation having excellent sliding properties, usage is possible in a wide array of fields in industrial applications, such as for automotive control cables or in-pipe work, and in medical applications, such as for catheters and guide wires. Also, the unevenness manufacturing method of the present invention may also be applied to electrical lines and coaxial cables. For example, by passing a metal wire through a resin tube, or by helically processing an electrical line having an outer diameter differential, an electrical line with excellent sliding properties in a shape similar to such a resin tube I is obtained. Furthermore, adding an outer conductor and sheath layer to the electrical line yields a coaxial cable. Since the coaxial cable has an undulation on the electrical line surface, or in other words on the insulation layer, gaps are formed between the insulator and the outer conductor, and improvements in characteristics as a low-permittivity coaxial cable are anticipated.

### Reference Signs List

- 1 to 4: resin tube
- 10: trunk section
- 12: outer surface
- 16: deep inner-core hole
- 20: trunk section
- 22: outer surface
- 24: outer surface
- 26: deep inner-core hole
- 30: trunk section
- 32 to 35: outer surface
- 36: deep inner-core hole
- 40: trunk section
- 42: outer surface
- 46: deep inner-core hole
- D10: maximum height difference
- D20: maximum height difference
- D30: maximum height difference
- D40: maximum height difference
- S10: cross-section
- S10a: cross-section
- S10b: cross-section
- S20: cross-section
- S30: cross-section
- S40: cross-section
- O1 to 04: axis of twisting
- SL: ridge line
- R1: maximum radius
- R2: minimum radius

## Claims

1. A resin tube (1) for a guide wire, the resin tube having a cross-sectional outer shape perpendicular to an axial direction that is polygonal, elliptic, or irregular, the resin tube being twisted in a helical shape about a deep inner-core hole (16) as an axis of twisting, the deep inner-core hole extending in the axial direction and allowing a core wire to be inserted therein, and the resin tube comprising a trunk section (10) having an undulation on the outer surface (12) thereof caused by the twisted eccentric wall thickness,
**characterized in that** the resin tube (1) is twisted such that the length of the pitch, being the distance in the axial direction over which one point on the outer circumference of a cross-sectional outer shape ofthe trunk section (10) is twisted 360 degrees in a helical shape and returns to the original position, becomes irregular in the axial direction of the trunk section (10).

2. The resin tube (1) for a guide wire according to Claim 1, **characterized in that** the height of a ridge line (SL) extending in the axial direction of the trunk section (10) varies along the axial direction.

3. The resin tube (1) for a guide wire according to Claim 1 or 2, **characterized in** being twisted such that the length of the pitch, being the distance in the axial direction over which one point on the outer circumference of a cross-sectional outer shape of the trunk section (10) is twisted 360 degrees in a helical shape and returns to the original position, is 6 times to 160 times a maximum radius R1, being the distance from the axis of twisting to a farthest point on the cross-sectional outer shape.

4. The resin tube (1) for a guide wire according to any one of Claims 1 to 3, **characterized in that** the relationship between a maximum radius R1, being the distance from the axis of twisting to a farthest point on the cross-sectional outer shape, and a minimum radius R2, being the distance from the axis of twisting to a nearest point on the cross-sectional outer shape, is 0.4 ≤ R2 / R1 ≤ 0.9.

5. A resin tube (1) for a guide wire, the resin tube having a cross-sectional outer shape perpendicular to an axial direction that is polygonal, elliptic, or irregular, the resin tube being twisted in a helical shape about a deep inner-core hole (16) as an axis of twisting, the deep inner-core hole extending in the axial direction and allowing a core wire to be inserted therein, and the resin tube comprising a trunk section (10) having an undulation on the outer surface (12) thereof caused by the twisted eccentric wall thickness,
**characterized in that**
the relationship between a maximum radius R1, being the distance from the axis of twisting to a farthest point on the cross-sectional outer shape, and a minimum radius R2, being the distance from the axis of twisting to a nearest point on the cross-sectional outer shape, is 0.4 ≤ R2 / R1 ≤ 0.9.

6. The resin tube (1) for a guide wire according to Claim 5, **characterized in that** the height of a ridge line (SL) extending in the axial direction of the trunk section varies along the axial direction.

7. The resin tube (1) for a guide wire according to Claim 5 or 6, **characterized in** being twisted such that the length of the pitch, being the distance in the axial direction over which one point on the outer circumference of a cross-sectional outer shape of the trunk section is twisted 360 degrees in a helical shape and returns to the original position, is 6 times to 160 times a maximum radius R1, being the distance from the axis of twisting to a farthest point on the cross-sectional outer shape.

8. The resin tube (1) for a guide wire according to any one of Claims 5 to 7, **characterized in** being twisted such that the length of the pitch, being the distance in the axial direction over which one point on the outer circumference of a cross-sectional outer shape of the trunk section (10) is twisted 360 degrees in a helical shape and returns to the original position, becomes irregular in the axial direction of the trunk section.

9. A method for manufacturing a resin tube (1) for a guide wire, comprising extruding a resin into a tube whose cross-sectional outer shape in the axial direction is polygonal, elliptic, or irregular, and having a deep inner-core hole extending in the axial direction and allowing a core wire to be inserted therein, and
twisting the extruded tube in a helical shape by a designated rotational amount about the deep inner-core hole as an axis of twisting,
**characterized by**
twisting the extruded tube in a helical shape by an irregular rotational amount.

10. The method for manufacturing a resin tube (1) for a guide wire according to Claim 9, **characterized by** twisting the extruded tube by a rotational amount such that the length of the pitch, being the distance in the axial direction over which one point on the outer circumference of the cross-sectional outer shape is twisted 360 degrees in a helical shape and returns to the original position, is 6 times to 160 times a maximum radius R1, being the distance from the axis of twisting to a farthest point on the cross-sectional outer shape.

11. A guide wire **characterized by** comprising
a resin tube (1) that has a cross-sectional outer shape perpendicular to an axial direction that is polygonal, elliptic, or irregular, is twisted in a helical shape about a deep inner-core hole (16) extending in the axial direction as an axis of twisting, and includes a trunk section (10) having an undulation on the outer surface (12) thereof caused by the twisted eccentric wall thickness, and
a core wire inserted into the deep inner-core hole (16) of the resin tube (1).

12. The guide wire according to Claim 11, **characterized in that** the height of a ridge line (SL) extending in the axial direction of the trunk section (10) of the resin tube varies along the axial direction.

13. The guide wire according to Claim1 or 12, **characterized in that** the resin tube (1) is twisted such that the length of the pitch, being the distance in the axial direction over which one point on the outer circumference of a cross-sectional outer shape of the trunk section (10) of the resin tube (1) is twisted 360 degrees in a helical shape and returns to the original position, is 6 times to 160 times a maximum radius R1, being the distance from the axis of twisting to a farthest point on the cross-sectional outer shape.

14. The guide wire according to any one of Claims 11 to 13, **characterized in that** the resin tube (1) is twisted such that the length of the pitch, being the distance in the axial direction over which one point on the outer circumference of a cross-sectional outer shape of the trunk section of the resin tube is twisted 360 degrees in a helical shape and returns to the original position, becomes irregular in the axial direction of the trunk section (10).

15. The guide wire according to anyone of Claims 11 to 14, **characterized in that** the relationship between a maximum radius R1, being the distance from the axis of twisting to a farthest point on the cross-sectional outer shape of the trunk section (10) of the resin tube (1), and a minimum radius R2, being the distance from the axis of twisting to a nearest point on the cross-sectional outer shape, is 0.4 ≤ R2 / R1 ≤ 0.9.

## Patentansprüche

1. Harzschlauch (1) für einen Führungsdraht, wobei der Harzschlauch eine zu einer axialen Richtung perpendikuläre Querschnittsaußenform aufweist, die vieleckig, elliptisch oder unregelmäßig ist, wobei der Harzschlauch um einen tiefliegenden Innenseelenhohlraum (16) als Verdrehachse schraubenförmig verdreht ist, wobei sich der tiefliegende Innenseelenhohlraum in der axialen Richtung erstreckt und das Einfügen einer Drahtseele darin ermöglicht, und wobei der Harzschlauch einen Rumpfabschnitt (10) mit einer Wellenform auf dessen Außenfläche (12) aufweist, die durch die verdrehte exzentrische Wanddicke verursacht ist, **dadurch gekennzeichnet, dass** der Harzschlauch (1) derart verdreht ist, dass die Ganghöhe, welche die Strecke in der axialen Richtung ist, über die ein Punkt auf dem Außenumfang einer Querschnittsaußenform des Rumpfabschnitts (10) um 360 Grad schraubenförmig verdreht ist und in die ursprüngliche Position zurückkehrt, in der axialen Richtung des Rumpfabschnitts (10) unregelmäßig wird.

2. Harzschlauch (1) für einen Führungsdraht nach Anspruch 1, **dadurch gekennzeichnet, dass** die Höhe einer Kammlinie (SL), die sich in der axialen Richtung des Rumpfabschnitts (10) erstreckt, längs der axialen Richtung variiert.

3. Harzschlauch (1) für einen Führungsdraht nach Anspruch 1 oder 2, **dadurch gekennzeichnet, dass** er derart verdreht ist, dass die Ganghöhe, welche die Strecke in der axialen Richtung ist, über die ein Punkt auf dem Außenumfang einer Querschnittsaußenform des Rumpfabschnitts (10) um 360 Grad schraubenförmig verdreht ist und in die ursprüngliche Position zurückkehrt, das 6- bis 160-fache eines Höchstradius R1 beträgt, welcher der Abstand von der Verdrehachse zu einem entferntesten Punkt auf der Querschnittsaußenform ist.

4. Harzschlauch (1) für einen Führungsdraht nach einem der Ansprüche von 1 bis 3, **dadurch gekennzeichnet, dass** das Verhältnis zwischen einem Höchstradius R1, welcher der Abstand von der Verdrehachse zu einem entferntesten Punkt auf der Querschnittsaußenform ist, und einem Mindestradius R2, welcher der Abstand von der Verdrehachse zu einem nächstgelegenen Punkt auf der Querschnittsaußenform ist, 0,4 ≤ R2 / R1 ≤ 0,9 ist.

5. Harzschlauch (1) für einen Führungsdraht, wobei der Harzschlauch eine zu einer axialen Richtung perpendikuläre Querschnittsaußenform aufweist, die vieleckig, elliptisch oder unregelmäßig ist, wobei der Harzschlauch um einen tiefliegenden Innenseelenhohlraum (16) als Verdrehachse schraubenförmig verdreht ist, wobei sich der tiefliegende Innenseelenhohlraum in der axialen Richtung erstreckt und das Einfügen einer Drahtseele darin ermöglicht, und wobei der Harzschlauch einen Rumpfabschnitt (10) mit einer Wellenform auf dessen Außenfläche (12) aufweist, die durch die verdrehte exzentrische Wanddicke verursacht ist, **dadurch gekennzeichnet, dass**
dass das Verhältnis zwischen einem Höchstradius R1, welcher der Abstand von der Verdrehachse zu einem entferntesten Punkt auf der Querschnittsaußenform ist, und einem Mindestradius R2, welcher der Abstand von der Verdrehachse zu einem nächstgelegenen Punkt auf der Querschnittsaußenform ist, 0,4 ≤ R2 / R1 ≤ 0,9 ist.

6. Harzschlauch (1) für einen Führungsdraht nach Anspruch 5, **dadurch gekennzeichnet, dass** die Höhe einer Kammlinie (SL), die sich in der axialen Richtung des Rumpfabschnitts erstreckt, längs der axialen Richtung variiert.

7. Harzschlauch (1) für einen Führungsdraht nach Anspruch 5 oder 6, **dadurch gekennzeichnet, dass** er derart verdreht ist, dass die Ganghöhe, welche die Strecke in der axialen Richtung ist, über die ein Punkt auf dem Außenumfang einer Querschnittsaußenform des Rumpfabschnitts um 360 Grad schraubenförmig verdreht ist und in die ursprüngliche Position zurückkehrt, das 6-bis 160-fache eines Höchstradius R1 beträgt, welcher der Abstand von der Verdrehachse zu einem entferntesten Punkt auf der Querschnittsaußenform ist.

8. Harzschlauch (1) für einen Führungsdraht nach einem der Ansprüche von 5 bis 7, **dadurch gekennzeichnet, dass** er derart verdreht ist, dass die Ganghöhe, welche die Strecke in der axialen Richtung ist, über die ein Punkt auf dem Außenumfang einer Querschnittsaußenform des Rumpfabschnitts (10) um 360 Grad schraubenförmig verdreht ist und in die ursprüngliche Position zurückkehrt, in der axialen Richtung des Rumpfabschnitts unregelmäßig wird.

9. Verfahren zur Herstellung eines Harzschlauchs (1) für einen Führungsdraht, das das Extrudieren eines Harzes zu einem Schlauch umfasst, dessen Querschnittsaußenform in der axialen Richtung vieleckig, elliptisch oder unregelmäßig ist und der einen tiefliegenden Innenseelenhohlraum aufweist, der sich in der axialen Richtung erstreckt und das Einfügen einer Drahtseele darin ermöglicht, und
Verdrehen des extrudierten Schlauchs mit einer Schraubenform um einen festgeigten Rotationsbetrag um den tiefliegenden Innenseelenhohlraum als Verdrehachse, **gekennzeichnet durch**
das Verdrehen des extrudierten Schlauchs mit einer Schraubenform um einen unregelmäßigen Rotationsbetrag.

10. Verfahren zur Herstellung eines Harzschlauchs (1) für einen Führungsdraht nach Anspruch 9, **dadurch gekennzeichnet, dass** der extrudierte Schlauch um einen derartigen Rotationsbetrag verdreht wird, dass die Ganghöhe, welche die Strecke in der axialen Richtung ist, über die ein Punkt auf dem Außenumfang der Querschnittsaußenform um 360 Grad schraubenförmig verdreht ist und in die ursprüngliche Position zurückkehrt, das 6- bis 160-fache eines Höchstradius R1 beträgt, welcher der Abstand von der Verdrehachse zu einem entferntesten Punkt auf der Querschnittsaußenform ist.

11. Führungsdraht, **dadurch gekennzeichnet, dass** er Folgendes umfasst:
einen Harzschlauch (1), der eine zu einer axialen Richtung perpendikuläre Querschnittsaußenform aufweist, die vieleckig, elliptisch oder unregelmäßig ist, und der schraubenförmig um einen sich in der axialen Richtung erstreckenden tiefliegenden Innenseelenhohlraum (16) als Verdrehachse verdreht ist und einen Rumpfabschnitt (10) mit einer Wellenform auf dessen Außenfläche (12) aufweist, die durch die verdrehte exzentrische Wanddicke verursacht ist, und
eine Drahtseele, die in den tiefliegenden Innenseelenhohlraum (16) des Harzschlauchs (1) eingefügt ist.

12. Führungsdraht nach Anspruch 11, **dadurch gekennzeichnet, dass** die Höhe einer Kammlinie (SL), die sich in der axialen Richtung des Rumpfabschnitts (10) des Harzschlauchs erstreckt, längs der axialen Richtung variiert.

13. Führungsdraht nach Anspruch 11 oder 12, **dadurch gekennzeichnet, dass** der Harzschlauch (1) derart verdreht ist, dass die Ganghöhe, welche die Strecke in der axialen Richtung ist, über die ein Punkt auf dem Außenumfang einer Querschnittsaußenform des Rumpfabschnitts (10) des Harzschlauchs (1) um 360 Grad schraubenförmig verdreht ist und in die ursprüngliche Position zurückkehrt, das 6- bis 160-fache eines Höchstradius R1 beträgt, welcher der Abstand von der Verdrehachse zu einem entferntesten Punkt auf der Querschnittsaußenform ist.

14. Führungsdraht nach einem der Ansprüche von 11 bis 13, **dadurch gekennzeichnet, dass** der Harzschlauch (1) derart verdreht ist, dass die Ganghöhe, welche die Strecke in der axialen Richtung ist, über die ein Punkt auf dem Außenumfang einer Querschnittsaußenform des Rumpfabschnitts des Harzschlauchs um 360 Grad schraubenförmig verdreht ist und in die ursprüngliche Position zurückkehrt, in der axialen Richtung des Rumpfabschnitts (10) unregelmäßig wird.

15. Führungsdraht nach einem der Ansprüche von 11 bis 14, **dadurch gekennzeichnet, dass** das Verhältnis zwischen einem Höchstradius R1, welcher der Abstand von der Verdrehachse zu einem entferntesten Punkt auf der Querschnittsaußenform des Rumpfabschnitts (10) des Harzschlauchs (1) ist, und einem Mindestradius R2, welcher der Abstand von der Verdrehachse zu einem nächstgelegenen Punkt auf der Querschnittsaußenform ist, 0,4 ≤ R2 / R1 ≤ 0,9 ist.

## Revendications

1. Tube en résine (1) destiné à un fil-guide, le tube en résine possédant une forme externe en coupe perpendiculaire à une direction axiale qui est polygonale, elliptique ou irrégulière, le tube en résine étant tordu en une forme hélicoïdale autour d'un trou de noyau interne profond (16) en tant qu'axe de torsion, le trou de noyau interne profond s'étendant dans la direction axiale et permettant à un fil de noyau d'être inséré en son sein, et le tube en résine comprenant une section de tronc (10) comportant une ondulation sur sa surface externe (12) provoquée par l'épaisseur de paroi excentrique tordue,
**caractérisé en ce que** le tube en résine (1) est tordu de telle sorte que la longueur du pas, qui est la distance dans la direction axiale sur laquelle un point sur la circonférence externe d'une forme externe en coupe de la section de tronc (10) est tordu à 360 degrés en une forme hélicoïdale et revient à la position originale, devient irrégulière dans la direction axiale de la section de tronc (10).

2. Tube en résine (1) destiné à un fil-guide selon la revendication 1, **caractérisé en ce que** la hauteur d'une ligne de crête (SL) s'étendant dans la direction axiale de la section de tronc (10) varie le long la direction axiale.

3. Tube en résine (1) destiné à un fil-guide selon la revendication 1 ou 2, **caractérisé en ce qu'**il est tordu de telle sorte que la longueur du pas, qui est la distance dans la direction axiale sur laquelle un point sur la circonférence externe d'une forme externe en coupe de la section de tronc (10) est tordu de 360 degrés en une forme hélicoïdale et revient à la position originale, vaut 6 fois à 160 fois un rayon maximum R1, qui est la distance allant de l'axe de torsion à un point le plus éloigné sur la forme externe en coupe.

4. Tube en résine (1) destiné à un fil-guide selon l'une quelconque des revendications 1 à 3, **caractérisé en ce que** la relation entre un rayon maximum R1, qui est la distance allant de l'axe de torsion à un point le plus éloigné sur la forme externe en coupe, et un rayon minimum R2, qui est la distance allant de l'axe de torsion à un point le plus proche sur la forme externe en coupe, vaut 0,4 ≤ R2 / R1 ≤ 0,9.

5. Tube en résine (1) destiné à un fil-guide, le tube en résine possédant une forme externe en coupe perpendiculaire à une direction axiale qui est polygonale, elliptique ou irrégulière, le tube en résine étant tordu en une forme hélicoïdale autour d'un trou de noyau interne profond (16) en tant qu'axe de torsion, le trou de noyau interne profond s'étendant dans la direction axiale et permettant à un fil de noyau d'être inséré en son sein, et le tube en résine comprenant une section de tronc (10) comportant une ondulation sur sa surface externe (12) provoquée par l'épaisseur de paroi excentrique tordue,
**caractérisé en ce que**
la relation entre un rayon maximum R1, qui est la distance allant de l'axe de torsion à un point le plus éloigné sur la forme externe en coupe, et un rayon minimum R2, qui est la distance allant de l'axe de torsion à un point le plus proche sur la forme externe en coupe, vaut 0,4 ≤ R2 / R1 ≤ 0,9.

6. Tube en résine (1) destiné à un fil-guide selon la revendication 5, **caractérisé en ce que** la hauteur d'une ligne de crête (SL) s'étendant dans la direction axiale de la section de tronc varie le long de la direction axiale.

7. Tube en résine (1) destiné à un fil-guide selon la revendication 5 ou 6, **caractérisé en ce qu'**il est tordu de telle sorte que la longueur du pas, qui est la distance dans la direction axiale sur laquelle un point sur la circonférence externe d'une forme externe en coupe de la section de tronc est tordu de 360 degrés en une forme hélicoïdale et revient à la position originale, vaut 6 fois à 160 fois un rayon maximum R1, qui est la distance allant de l'axe de torsion à un point le plus éloigné sur la forme externe en coupe.

8. Tube en résine (1) destiné à un fil-guide selon l'une quelconque des revendications 5 à 7, **caractérisé en ce qu'**il est tordu de telle sorte que la longueur du pas, qui est la distance dans la direction axiale sur laquelle un point sur la circonférence externe d'une forme externe en coupe de la section de tronc (10) est tordu à 360 degrés en une forme hélicoïdale et revient à la position originale, devient irrégulière dans la direction axiale de la section de tronc.

9. Procédé de fabrication d'un tube en résine (1) destiné à un fil-guide, comprenant l'extrusion d'une résine en un tube dont la forme externe en coupe dans la direction axiale est polygonale, elliptique ou irrégulière, et comportant un trou de noyau interne profond s'étendant dans la direction axiale et permettant à un fil de noyau d'être inséré en son sein, et
la torsion du tube extrudé en une forme hélicoïdale selon une valeur de rotation désignée autour du trou de noyau interne profond en tant qu'axe de torsion, **caractérisé par**
la torsion du tube extrudé en une forme hélicoïdale selon une valeur de rotation irrégulière.

10. Procédé de fabrication d'un tube en résine (1) destiné à un fil-guide selon la revendication 9, **caractérisé par** la torsion du tube extrudé selon une valeur de rotation telle que la longueur du pas, qui est la distance dans la direction axiale sur laquelle un point sur la circonférence externe de la forme externe en coupe est tordu de 360 degrés en une forme hélicoïdale et revient à la position originale, vaut 6 fois à 160 fois un rayon maximum R1, qui est la distance allant de l'axe de torsion à un point le plus éloigné sur la forme externe en coupe.

11. Fil-guide **caractérisé en ce qu'**il comprend un tube en résine (1) qui possède une forme externe en coupe perpendiculaire à une direction axiale qui est polygonale, elliptique ou irrégulière, qui est tordu en une forme hélicoïdale autour d'un trou de noyau interne profond (16) s'étendant dans la direction axiale en tant qu'axe de torsion, et comprend une section de tronc (10) comportant une ondulation sur sa surface externe (12) provoquée par l'épaisseur de paroi excentrique tordue, et
un fil de noyau inséré dans le trou de noyau interne profond (16) du tube en résine (1).

12. Fil-guide selon la revendication 11, **caractérisé en ce que** la hauteur d'une ligne de crête (SL) s'étendant dans la direction axiale de la section de tronc (10) du tube en résine varie le long de la direction axiale.

13. Fil-guide selon la revendication 11 ou 12, **caractérisé en ce que** le tube en résine (1) est tordu de telle sorte que la longueur du pas, qui est la distance dans la direction axiale sur laquelle un point sur la circonférence externe d'une forme externe en coupe de la section de tronc (10) du tube en résine (1) est tordu de 360 degrés en une forme hélicoïdale et revient à la position originale, vaut 6 fois à 160 fois un rayon maximum R1, qui est la distance allant de l'axe de torsion à un point le plus éloigné sur la forme externe en coupe.

14. Fil-guide selon l'une quelconque des revendications 11 à 13, **caractérisé en ce que** le tube en résine (1) est tordu de telle sorte que la longueur du pas, qui est la distance dans la direction axiale sur laquelle un point sur la circonférence externe d'une forme externe en coupe de la section de tronc du tube en résine est tordu de 360 degrés en une forme hélicoïdale et revient à la position originale, devient irrégulière dans la direction axiale de la section de tronc (10).

15. Fil-guide selon l'une quelconque des revendications 11 à 14, **caractérisé en ce que** la relation entre un rayon maximum R1, qui est la distance allant de l'axe de torsion d'un point le plus éloigné sur la forme externe en coupe de la section de tronc (10) du tube en résine (1), et un rayon minimum R2, qui est la distance allant de l'axe de torsion à un point le plus proche sur la forme externe en coupe, vaut 0,4 ≤ R2 / R1 ≤ 0,9.
